(19) Europäisches Patentamt
European Patent Office
Office européen des brevets

(11) Veröffentlichungsnummer : **0 477 499 B1**

(12) # EUROPÄISCHE PATENTSCHRIFT

(45) Veröffentlichungstag der Patentschrift :
**26.01.94 Patentblatt 94/04**

(51) Int. Cl.$^5$ : **C07K 7/20,** A61K 37/43

(21) Anmeldenummer : **91112817.1**

(22) Anmeldetag : **30.07.91**

(54) **Gonadoliberin-Antagonisten.**

(30) Priorität : **04.08.90 DE 4024779**

(43) Veröffentlichungstag der Anmeldung :
**01.04.92 Patentblatt 92/14**

(45) Bekanntmachung des Hinweises auf die Patenterteilung :
**26.01.94 Patentblatt 94/04**

(84) Benannte Vertragsstaaten :
**AT BE CH DE DK ES FR GB GR IT LI LU NL SE**

(56) Entgegenhaltungen :
**EP-A- 0 263 521**
**WO-A-89/09786**

(56) Entgegenhaltungen :
**BIOCHEMICAL & BIOPHYSICAL RESEARCH COMMUNICATIONS,Band 148,Nr 2,29. Oktober 1987,Seiten 849-856,Academic Press,Inc.,-Duluth,MN,US;A.LJUNQVIST et al.:"Design,synthesis and bioassays of antagonists of LHRH which have high antiovulatory activity and release negligible histamine"**

(73) Patentinhaber : **HOECHST AKTIENGESELLSCHAFT**
**D-65926 Frankfurt (DE)**

(72) Erfinder : **König, Wolfgang, Dr.**
**Eppsteiner Strasse 25**
**W-6238 Hofheim/Ts. (DE)**
Erfinder : **Sandow, Jürgen, Dr.**
**Am Heideplacken 22**
**W-6240 Königstein/Ts. (DE)**
Erfinder : **Kolar, Cenek, Dr.**
**Deutschhausstrasse 20**
**W-3550 Marburg (DE)**

EP 0 477 499 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gonadoliberin-Antagonisten

Natürlich vorkommende Gonadoliberine (Gn-RH) verschiedener Spezies sind Dekapeptide folgender Strukturen:

| | |
|---|---|
| h-,p-,o- | Pgl-His-Trp-Ser-Tyr-Gly-Leu-Arg-Pro-Gly-$NH_2$ |
| g-Gn-RH-I | Pgl-His-Trp-Ser-Tyr-Gly-Leu-Gin-Pro-Gly-$NH_2$ |
| g-Gn-RH-II | Pgl-His-Trp-Ser-His-Gly-Trp-Tyr-Pro-Gly-$NH_2$ |
| sa-Gn-RH | Pgl-His-Trp-Ser-Tyr-Gly--Trp-Leu-Pro-Gly-$NH_2$ |
| pe-Gn-RH | Pgl-His-Tyr-Ser-Leu-Glu-Trp-Lys-Pro-Gly-$NH_2$ |

[h- (Mensch), p- (Schwein), o- (Schaf): Biochem. Biophys. Res. Commun. 43(1971) 1334; g- (Huhn-I): South Africa J. Science 78 (1982) 124; g- (Huhn-II): Proc. Natl. Acad. Sci. USA 81 (1984) 3874; sa(Lachs): Proc. Natl. Acad. Sci. USA 80 (1983) 2794; pe- (Neunauge): J. Biol. Chem. 261 (1986) 4812-4819.]

Gn-RH wird bei Säugern hauptsächlich im Hypothalamus gebildet und bewirkt in der Hypophyse eine Ausschüttung von Lutropin (LH) und Follitropin (FSH).

Kompetitive Antagonisten des Gn-RH hemmen über die Blockierung der Gn-RH-Rezeptoren die Bildung von LH und FSH und damit auch die Synthese von Östrogen bei weiblichen Tieren bzw. Frauen oder Testosteron bei männlichen Tieren bzw. Männern. In der Literatur wurden bereits zahlreiche Gn-RH-Antagonisten beschrieben [J.J. Nestor, Jr. et al. In: LH-RH and its analogues (F. Labrie et al., eds.) Elsevier Science Publishers B.V. 1984. pp. 24-35; A.S. Dutta, Drugs of the Future 13 (1986) 761-787.], die meist eine basische Aminosäure in Position 6 enthalten. Durch diese basische Ladung in Position 6 werden die Peptide wasserlöslicher. Eine negative Begleiterscheinung dieser basischen Gruppe ist jedoch eine Histamin-ausschüttende Wirkung. Das "Nal-Glu", bei dem das Arg in Position 5 versetzt wurde und in Position 6 D-4-p-Methoxybenzoyl-2-amino-buttersäure steht, hat eine stark reduzierte Histaminausschüttung [A. Phillips et al., Life Sci. 41 (1987) 2017-2022]. Geringer basische Substitutionen in Position 6, wie z.B. D-Nicotinoyl-Lysin [K Folkers et al., Z. Naturforsch. 42b (1987) 101-106; A. Ljungqvist et al., Biochem. Biophys. Res. Commun. 148 (1987) 849-856]. D-Citrullin oder D-Homocitrullin [S. Bajusz et al. Proc. Natl. Acad. Sci. USA 85 (1988) 1637-1641] verringern ebenso die Histamin-Freisetzung.

In der EP-A 263 521 (HOE 86/F 253) wurde durch Substitution mit glycosylierten Zuckern sowohl Gn-RH-Agonisten als auch Gn-RH-Antagonisten mit günstigen Eigenschaften erhalten.

Zum einen konnte die Wasserlöslichkeit erhöht werden und zum anderen die anaphylaktische Wirkung, die vor allem bei Gn-RH-Antagonisten zu beobachten war, gesenkt werden.

In der WO-A-89/09786 werden LHRH-Antagonisten-Peptidderivate beschrieben, die Zuckerreste oder kurze Polyhydroxyreste enthalten.

Durch Ersatz des Arginins in Position 8 durch andere basische Aminosäuren wie Nε-Isopropyl-L-Lysin [A. Ljungqvist et al., Biophys. Res. Commun. 148 (1987) 849-856] oder Ng,Ng'-Diethyl-L-Homoarginin [C.-H. Less et al. Life Sci. 45 (1989) 697-702] konnte die Histamin-Freisetzung unter Erhalt der- antagonistischen Wirkung gesenkt werden. Bisher wurde davon ausgegangen, daß für die Rezeptorbindung des Gn-RH bei Säugetieren die basische Ladung in Position 8 eine wichtige Rolle spielt (E. Hazum und P.M. Conn, Endocrine Reviews 9 (1988) 379-386).

Die Aufgabe, die Histaminfreisetzung unter Erhalt der antagonistischen Wirkung weiter zu senken, wird überraschenderweise dadurch gelöst, daß gegenüber den bekannten, in 8 Position eine basische Ladung enthaltenden Gn-RH-Derivaten, diese Position durch glycosyliertes L-Serin ausgetauscht ist.

Die Erfindung betrifft Peptide der allgemeinen Formel I,

$$\begin{matrix} & 1 & & 2 & & 3 & & 4 & & & 5 & & 6 & & 7 & & 8 & & 9 & & 10 \\ X & - & A & - & B & - & C & - & Ser & - & D & - & E & - & F & - & G & - & Pro & - & H \end{matrix} \quad (I),$$

in welcher

X ($C_2$-$C_8$)-Alkanoyl bedeutet;

A D-Nal (2), D-Phe oder D-Trp bedeutet, wobei der Aromat gegebenenfalls durch einen oder zwei gleiche oder verschiedene Reste aus der Reihe Brom, Chlor, Fluor, Nitro, $C_1$-$C_4$-Alkyl, insbesondere Methyl, und $C_1$-$C_4$-Alkoxy, insbesondere Methoxy, substituiert sein kann;

B D-Phe bedeutet, das gegebenenfalls durch einen oder zwei gleiche oder verschiedene Reste aus der Reihe Brom, Chlor, Fluor, Nitro, Amino, $C_1$-$C_4$-Alkyl, insbesondere Methyl, und $C_1$-$C_4$-Alkoxy, insbeson-

dere Methoxy, substituiert sein kann;

C D-Pal (3), D-Phe oder D-Trp bedeutet, wobei der Aromat von D-Phe und D-Trp gegebenenfalls durch einen oder zwei gleiche oder verschiedene Reste aus der Reihe Brom, Chlor, Fluor, Nitro, $C_1$-$C_4$-Alkyl, insbesondre Methyl, und $C_1$-$C_4$-Alkoxy, insbesondere Methoxy, substituiert sein kann;

D Tyr oder His bedeutet;

E D-Ser($R^1$) bedeutet;

F Leu, Trp oder Phe bedeutet;

G L-Ser($R^1$) bedeutet;

H Gly-$NH_2$, D-Ala-$NH_2$ oder Azagly-$NH_2$ bedeutet;

$R^1$ einen Glycosylrest bedeutet;

sowie deren physiologisch verträgliche Salze.

Bevorzugt sind Gn-RH-Antagonisten der allgemeinen Formel I, in welcher

X Acetyl;

A D-Nal(2);

B D-Phe(p-Cl);

C D-Pal(3) oder D-Trp;

D Tyr;

E D-Ser($R^1$);

F Leu;

G L-Ser($R^1$);

Tieren oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetyl-galactosamin (GalNAc), N-Acetyl-Mannosamin (ManNAc) oder Disacchariden, wie Maltose (Mal), Lactose (Lac), Cellobiose (Cel), Gentobiose (Gen), N-Acetyl-Lactosamin (LacNAc), Chitobiose (Chit), β-Galactopyranosyl-(1-3)- oder -(1-4)-N-acetyl-glucosamin, sowie deren synthetischen Derivate, wie 2-Desoxy-, 2-Amino-, 2-Acetamido- oder 2-Halogeno-, bevorzugt Bromo- und Jodo-Zucker ab.

Falls nicht anders angegeben stehen die Aminosäuren ohne Stereodescriptor für L-Aminosäuren. Unter physiologisch verträglichen Salzen versteht man insbesondere solche mit anorganische Säuren, wie HCl, HBr, $H_2SO_4$, $H_3PO_4$, oder organischen Säuren, wie Essigsäure, Maleinsäure, Fumarsäure, Weinsäure, Citronensäure.

Die Erfindung betrifft ferner ein Verfahren zur Herstellung der Peptide der allgemeinen Formel I, das dadurch gekennzeichnet ist, daß man ein Fragment mit N-terminaler freier Aminogruppe mit einem Fragment mit C-terminaler freier Carboxylgruppe kondensiert, eine oder mehrere zum Schutz funktioneller Gruppen gegebenenfalls temporär eingeführte Schutsgruppen abspaltet und das so erhaltene Peptid gegebenenfalls in sein physiologisch verträgliches Salz überführt.

Die Auswahl der Schutzgruppen und die Synthesestrategie wird von der Art und Konfiguration der Aminosäuren sowie der Art der Kupplungsbedingungen bestimmt. Geeignete Methoden sind beispielsweise in EP-A 263 521 beschrieben bzw. es sind die allgemeinen Methoden der Peptidchemie (Houben-Weyl, Methoden der Organischen Chemie, Band 125) durch stufenweisen Aufbau vom C-terminalem Ende oder die Segment-Kondensation. Die Synthese der Serin-glycoside ist in EP-A 263 521 beschrieben.

Um bei der Segmentkondensation die mögliche Racemisierung möglichst niedrig zu halten wird hier bevorzugt mit Dicyclohexylcarbodiimid (DCC) unter Zusatz von 3-Hydroxy-4-oxo-3,4-dihydro-1,2,3-benzotriazin (HOObt) gearbeitet. Als Amino-Schutzgruppen werden bevorzugt der durch katalytische Hydrierung abspaltbare Z-Rest oder der durch sekundäre Amine abspaltbare Fmoc-Rest herangezogen.

Als besonders günstig erwies sich eine Segment-Kupplung nach dem Schema

$$(1\text{-}5) + (6\text{-}10) \rightarrow (1\text{-}10).$$

H D-Ala-$NH_2$ oder Azagly-$NH_2$ bedeuten,

sowie deren physiologisch verträgliche Salze.

Alkyl bzw. Alkoxy kann geradkettig oder verzweigt sein.

$R^1$ ist vorzugsweise ein Glycosylrest, der sich von einer Glycopyranose, Glycofuranose oder einem Oligosaccharid ableitet. Die Glycosylreste können sowohl α- als auch β-glycosidisch mit dem Serin-Rest verknüpft sein.

$R^1$ kann beispielsweise ein Glucofuranosyl- oder Glucopyranosyl-Rest sein, der sich von natürlich vorkommenden Aldotetrosen, Aldopentosen, Oligosacchariden, wie Di- und Trisacchariden, sowie deren Stereoisomeren ableiten.

Diese Glycosylreste R[1] leiten sich insbesondere von natürlichen, in Mikroorganismen, Pflanzen, Die Synthese wird durch nachfolgendes Reaktionsschema illustriert.

SCHEMA 1

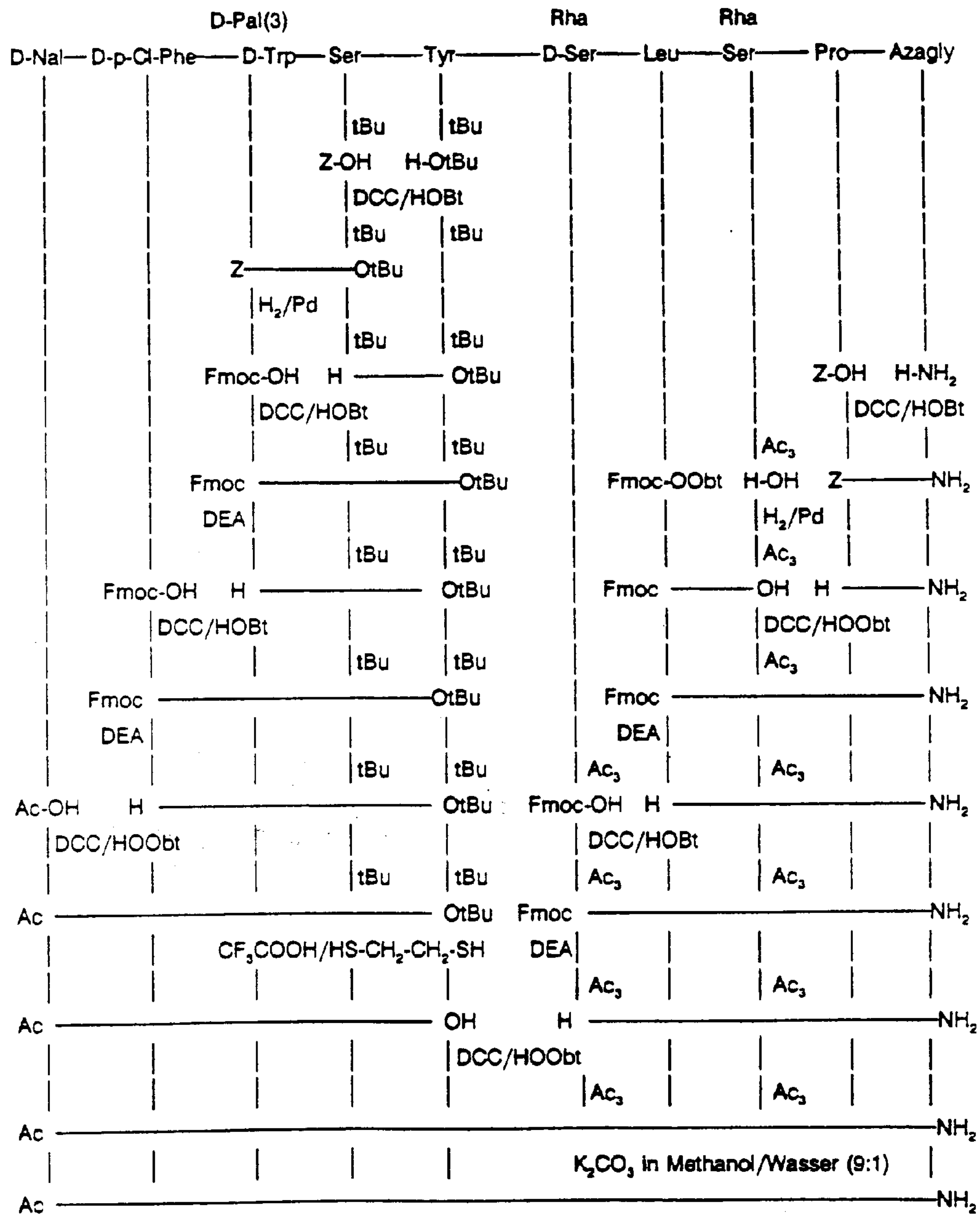


Die erfindungsgemäßen Gn-RH Antagonisten wirken hemmend auf die Bildung der Gonadotropine

Lutropin und Follitropin und damit auch auf die Synthese von Testosteron und Östrogen. Sie können wie hochdosierte Gn-RH-Agonisten bei Gonadotropin- und Steroid-abhängigen Erkrankungen eingesetzt werden, wie beispielsweise in EP-A 263 521 beschrieben, sowie bei der Geburtenkontrolle; Pubertas precox; bei der Behandlung von Testosteron- und Östrogen-abhängigen Tumoren wie z.B. Prostatakrebs und Brustkrebs; bei der Behandlung von Endometriose und Myomen. Ferner schützten die erfindungsgemäßen Peptide die Keimdrüsen von Röntgenstrahlung. Der Vorteil der Antagonisten gegenüber den Agonisten ist jedoch, daß die primäre Stimulierungsphase der Agonisten vermieden wird.

Die erfindungsgemäßen Gn-RH-Antagonisten können parenteral, intranasal oder als Implantate appliziert werden wie beispielsweise in EP-A 263 521 beschrieben. Die bevorzugten Anwendungsformen beim Menschen sind die intranasale Applikation oder die Verwendung von Implantaten.

Mittels eines Dosierzerstäubers werden über eine Spraydüse etwa 0,02-0,2 ml einer Pufferlösung, in der die erforderliche Menge des Wirkstoffs gelöst ist, in die Nase gesprüht. Bei einer parenteralen Applikation können die Dosierungen gegenüber der Intranasal-Dosis um etwa eine Zehnerpotenz gesenkt werden.

Die erfindungsgemäßen Antagonisten werden beim erwachsenen Menschen intranasal in Dosen von 1-10 mg appliziert. Die Einzeldosis in Implantaten beträgt etwa 5-50 mg für einen Zeitraum von jeweils 4-8 Wochen. Parenteral appliziert genügen bereits 0,1-1 mg/Tag.

Die erfindungsgemäßen Peptide wurden an männlichen Ratten mittels Dauerinfusion (MINIPUMPS) auf athrophische Wirkung an androgenabhängigen Organen und auf LH- und Testosteron-senkende Wirkung im Serum und Blut geprüft. An weiblichen Ratten wurde die ovulationshemmende Wirkung getestet. Die Histaminfreisetzung wurde an Ratten-Peritonealmastzellen überprüft.

Testosteron-senkende Wirkung:

Die Testosteron-senkende Wirkung wurde getestet in männlichen Ratten während Serumtestosteron wurde mit Diethylether extrahiert und mittels spezifischem RIA bestimmt.

Testosteron-senkende Wirkung durch Minipumpen-Infusion

| Beispiel | Dosis pro Tag ($\mu$g/24 h/Ratte) |
|---|---|
| 1 | 120 |
| 2 | 120 |
| 3 | 120 |
| 4 | 60 |
| 5 | 60 |

Ovulationshemmende Wirkung:

Ovulationshemmende Wirkung wurde in unreifen weiblichen Ratten getestet die mit PMSG (Pregnant Mare Serum Gonadotropin) vorbehandelt wurden, um Follikelreifung zu induzieren. Spontane Ovulation wurde verhindert durch Barbiturat (Phenobarbital). Eine Testdosis von 800 ng LHRH wurde 2 Stunden nach den Antagonisten subkutan injiziert. Die Testsubstanzen gelöst in 5 %iger Mannit-Lösung, wurden subkutan injiziert. Am nächsten Tag wurden die Eileiter herausseziert und mit Patentblau gefärbt, die röhrenförmigen Eizellen wurden unter dem Mikroskop gezählt. Die Dosis der ovulationshemmenden Wirkung aller Antagonisten (ED 100) wurde bestimmt.

Ratten Antiovulations-Assay

| Beispiel | Ovulationshemmende Dosis (ng pro Ratte s.c.) |
|---|---|
| 1 | 120 |
| 2 | 256 |
| 3 | 512 |
| 4 | 64 |
| 5 | 64 |

Histaminfreisetzung:

a) Zubereitung der peritonealen Zellsuspension

Wistarratten wurden durch Enthauptung getötet. 50 ml einer 0,9 %igen NaCl-Lösunng wurden in subkutaner Infusion über 7 Tage. Der biologische Effekt auf Androgen-abhängigen Organen und Serumtestosteron wurde aufgezeichnet. Die Testverbindungen wurden durch subkutane Infusion mittels Minipumpen bei einer Rate von 30 - 120 µg/Tag 7 Tage lang verabreicht. Die Testverbindungen wurden in steriler, 5%iger Mannitlösung gelöst. Die Minipumpen werden subkutan in der Gegend des Rückens unter Narkose und aseptischen Bedingungen implantiert.

die Bauchhöhle injiziert und nach leichter Massage die Bauchdecke geöffnet. Die Flüssigkeit, die die peritonealen Zellen enthält, wurde mittels einer Pasteurpipette abgesaugt und anschließend zentrifugiert. Die abgesetzten Zellen wurden wieder suspendiert und nach Prüfung auf Lebensfähigkeit auf Mastzellen/ml verdünnt.

b) Behandlung der Mastzellen

150 µl des gelösten Gonadoliberin-Antagonisten wurde zu einer Suspension von $10^5$ Mastzellen in 150 µl 0,9 %iger NaCl-Lösung gegeben. Nach 30 minütiger Inkubation bei 37 °C wurden die Teströhren zentrifugiert und der Überstand abgenommen. Der LHRH-Antagonist Ac-D-Nal-D-P-Cl-D-Phe-D-Trp-Ser-Tyr-D-Ser(Rha)-Leu-Arg-Pro-Azagly-$NH_2$, bekannt als eine Verbindung die die Histaminfreisetzung von Mastzellen induziert wurde als Vergleichssubstanz verwendet. Zur Bestimmung der totalen Histaminfreisetzung (100 %) wurden die Mastzellensuspensionen rasch über einem Bunsenbrenner gekocht.

c) HPLC-Bestimmung der Histaminlevel

Eine modifizierte Form der von Skofitch et al. (J. Chromatogrophy, 226, 53 - 59, 1981) und Siraganian und Hook (in Manual of Clinical Laboratory Immunology; eds: Rose et al. American Society of Microbiology, Washington D. C., Seiten 675, 1986) beschriebenen Methoden wurde verwendet. Kurz zusammengefaßt wurde zu 250 µl Überstand 100 µl einer 1N NaOH und 100 µl Phthaldialdehyd zugegeben und stark geschüttelt. Nach 2 Minuten Reaktionszeit wurde das Fluorphor in ein stärker fluoreszierendes und stabiles Produkt durch Ansäuern mit 50 µl 3N HCl umgewandelt. Vom Überstand wurden 10 µl auf die Chromspher C8 Kolonne (Chrompak FRW) des HPLC-Systems injiziert. Dieses System besteht aus einem Chromatograph SP 8100 (Spectra Physics) und einem Integrator SP 4270 (Spectra Physics) und einem Fluoreszens-Detektor LS-1 (Perkin Elmer). Die Fluoreszens wurde aufgezeichnet bei 360 nm Excitation und 450 nm Emissionwellenlängen.

Histaminfreisetzung von peritonealen Mastzellen (µg/ml)

(Ergebnisse sind in Prozenten relativ zu den Gesamtwerten der Histaminfreisetzung bedingt durch die Lysis der Mastzellen beim Kochen gegeben)

| Beispiel | 100 µg/ml | 10 µg/ml | 1 µg/ml | 0,1 µg/ml | 0,01 µg/ml |
|---|---|---|---|---|---|
| 1 | -8,61 | -5,37 | -2,16 | 2,82 | 0,33 |
| 2 | 0,0 | 0,0 | 3,7 | 4,6 | 2,5 |
| 5 | 3,8 | 0,3 | 0,9 | 2,4 | 1,9 |
| Vergleichs beispiel | 107,6 | 92,4 | 15,4 | 2,1 | 2,6 |

Weitere verwendete Abkürzungen:

| | |
|---|---|
| DCC | Dicyclohexylcarbodiimid |
| DEA | Diethylamin |
| HOBt | 1-Hydroxybenzotriazol |
| MTB-Ether | Methyl-tert.-butyl-ether |
| Nal | 3-(2-Naphthyl)-alanin |
| Pal | 3-(3-Pyridyl)-alanin |

Nachfolgende Beispiele erläutern die vorliegende Erfindung.

1. Beispiel

Ac-D-Nal-D-p-Cl-Phe-D-Trp-Ser-Tyr-D-Ser(Rha)-Leu-Ser(Rha)-Pro-Azagly-$NH_2$

1a. H-Ser[Rha($Ac_3$)]-OH

Zu einer Lösung von 30 g (50 mmol) Fmoc-Ser[Rha($Ac_3$)]-OH in 150 ml Dimethylformamid gibt man unter Rühren 55 ml Diethylamin. Nach 10 Minuten wird die Lösung im Hochvakuum eingeengt und der Rückstand mit MTB-Ether verrieben. Man saugt den Niederschlag ab und wäscht mit MTB-Ether nach. Die Substanz wird im Hochvakuum getrocknet.

Ausbeute: 17,13 g (91 %)

$[\alpha]_D^{26}$ = -31° (c=1, in Eisessig)

1b. Fmoc-Leu-Ser[Rha($Ac_3$)]-OH

Zu einer Suspension von 17 g (45 mmol) H-Ser[Rha($Ac_3$)]-OH in 100 ml Dimethylformamid gibt man bei 0 °C und unter Rühren 20,4 g Fmoc-Leu-OObt. Nach 1 Stunde hat sich alles gelöst. Man läßt über Nacht bei Raumtemperatur stehen und engt im Hochvakuum ein. Der Rückstand wird in Essigester gelöst und nacheinander 3 mal mit gesättigter $NaHCO_3$-Lösung, Wasser, 2 mal mit $KHSO_4$-Puffer und 2 mal mit Wasser ausgeschüttelt. Die Essigesterphase wird über $Na_2SO_4$ getrocknet, eingeengt und der Rückstand aus Diethylether/Petrolether umgefällt. Der Niederschlag wird nochmal mit Petrolether digeriert und abgesaugt.

Ausbeute: 22,2 g (69 %)

$[\alpha]_D^{26}$ = -20° (c=1, in Eisessig)

1c. Fmoc-Leu-Ser[Rha($Ac_3$)]-Pro-Azagly-$NH_2$

Zu einer Lösung von 5 g $HClO_4 \cdot$H-Pro-Azagly-$NH_2$ (16,4 mmol), 11,7 g Fmoc-Leu-Ser[Rha($Ac_3$)]-OH und 2,7 g HOObt in 60 ml Dimethylformamid gibt man bei 0 °C unter Rühren 2 ml N-Ethylmorpholin und 3,5 g DCC. Nach 1 Stunde Rühren bei 0 °C stellt man über Nacht bei Raumtemperatur. Anderntags wird der Niederschlag abgesaugt und das Filtrat eingeengt. Der Rückstand wird in Essigester gelöst und nacheinander mit Wasser, 2 mal mit gesättigter $NaHCO_3$-Lösung, Wasser, 2 mal mit $KHSO_4$-Puffer und Wasser gewaschen, über $Na_2SO_4$ getrocknet. Die Lösung wird im Vakuum eingeengt und der Rückstand mit Diethylether verrieben.

Ausbeute: 11,87g (83 %)

$[\alpha]_D^{26}$ = -54,8° (c=1, in Eisessig)

1d. H-Leu-Ser[Rha($Ac_3$)]-Pro-Azagly-$NH_2$

Zu einer Lösung von 11,9 g (13,7 mmol) Fmoc-Leu-Ser[Rha($Ac_3$]-Pro-Azagly-$NH_2$ in 90 ml Dimethyl-

formamid gibt man unter Rühren bei Raumtemperatur 15 ml Diethylamin. Nach 10 Minuten engt man im Hochvakuum ein und verreibt den Rückstand mit Diethylether. Der Niederschlag wird abgesaugt und mit MTB-Ether gewaschen.

Ausbeute: 7,71 g (87 %)

1e. Fmoc-D-Ser[Rha($Ac_3$)]-Leu-Ser[Rha($Ac_3$)]-Pro-Azagly-$NH_2$

Zu einer Lösung von 7,71 g (12 mmol) H-Leu-Ser[Rha($Ac_3$)]-Pro-Azagly-$NH_2$, 7,2 g Fmoc-D-Ser[Rha($Ac_3$)] und 1,68 g HOBt in 60 ml Dimethylformamid gibt man unter Rühren bei 0 °C 2,52 g DCC. Man läßt 1 Stunde bei 0 °C rühren und über Nacht bei Raumtemperatur stehen. Man arbeitet wie in Beispiel 1c auf. Zur weiteren Reinigung wird die Substanz auf Kieselgel in Methylenchlorid/Aceton (9:1) und Methylenchlorid/Methanol (9:0,5) chromatographiert.

Ausbeute: 11,55 g (78 %)

1f. H-D-Ser[Rha($Ac_3$)]-Leu-Ser[Rha($Ac_3$)]-Pro-Azagly-$NH_2$

Zu einer Lösung von 4,75 g (3,87 mmol) Fmoc-D-Ser[Rha($Ac_3$)]-Leu-Ser[Rha($Ac_3$)]-Pro-Azagly-$NH_2$ in in 30 ml Dimethylformamid gibt man bei Raumtemperatur unter Rühren 4,2 ml Diethylamin. Nach 10 Minuten wird ein Hochvakuum eingeengt und der Rückstand mit Diethylether 2 mal verrieben.

Ausbeute: 3,46 g (89 %)

$[\alpha]_D^{26}$ = -63° (c=2, in Eisessig)

1g. Z-Ser(tBu)-Tyr(tBu)-OtBu

44,7 g (151,4 mmol) Z-Ser(tBu)-OH, 49,9 g (151,3 mmol) H-Tyr(tBu)-OtBu·HCl und 20,4 g (151,1 mmol) HOBt werden in 200 ml Dimethylformamid gelöst. Bei 0 °C gibt man unter Rühren 19,4 ml (151,6 mmol) N-Ethylmorpholin und 33,3 g (151,4 mmol) DCC zu. Man läßt 1 Stunde bei 0 °C rühren und über Nacht bei Raumtemperatur stehen. Der Niederschlag wird abgesaugt und das Filtrat eingeengt. Der Rückstand wird in Essigester gelöst und nacheinander mit Wasser, $KHSO_4/K_2SO_4$-Puffer, $NaHCO_3$-Lösung und Wasser extrahiert, über $Na_2SO_4$ getrocknet und eingeengt.

Ausbeute: 87 g Öl (100,8 %)

1h. H-Ser-(tBu)-Tyr(tBu)-OtBu·HCl

87,3 g (153 mmol) Z-Ser(tBu)-Tyr(tBu)-OtBu werden in 600 ml Methanol gelöst und mit Pd/C-Katalysator versetzt. Am Autotitrator wird unter Zugabe von methanolischer Salzsäure bei pH 4,5 und Durchleiten von Wasserstoff hydriert. Nach beendeter Hydrierung wird der Katalysator über Kieselgur abgesaugt, das Filtrat eingeengt und der Rückstand mit Petrolether verrieben. Es wird abgesaugt und über $P_2O_5$ im Hochvakuum getrocknet.

Ausbeute: 55,8 g (77 %)

$[\alpha]_D^{26}$ = +1,6° (c=1, in Methanol)

Schmp. 109-111 °C

1i. Fmoc-D-Trp-Ser(tBu)-Tyr(tBu)-OtBu

48,5 g (102,5 mmol) H-Ser(tBu)-Tyr(tBu)-OtBu·HCl, 43,75 g (102,5 mmol) Fmoc-D-Trp-OH und 13,83 g (102,4 mmol) HOBt werden in 200 ml Dimethylformamid gelöst. Bei 0 °C gibt man unter Rühren 13,1 ml (102,3 mmol) N-Ethylmorpholin und 22,55 g (102,5 mmol) DCC zu. Man läßt 1 Stunde bei 0 °C rühren und über Nacht bei Raumtemperatur stehen. Danach wird der Niederschlag abgesaugt und das Filtrat eingeengt. Der Rückstand wird in Essigester gelöst und nacheinander mit Wasser, $KHSO_4/K_2SO_4$-Puffer, $NaHCO_3$-Lösung und Wasser ausgeschüttet, über $Na_2SO_4$ getrocknet und eingeengt.

Ausbeute: 93,0 g Öl (107 %, enthält noch DC-Harnstoff)

1k. H-D-Trp-Ser(tBu)-Tyr(tBu)-OtBu

93,0 g (ca. 102 mmol) Fmoc-D-Trp-Ser(tBu)-Tyr(tBu)-OtBu werden in 500 ml Dimethylformamid gelöst. Dazu gibt man unter Rühren 114,5 ml Diethylamin, läßt 10 Minuten bei Raumtemperatur reagieren und engt im Hochvakuum ein. Der Rückstand wird 3 mal mit Petrolether und 2 mal mit Diethylether verrieben. Nach Abdestillieren des Diethylethers wird zwischen Essigester und Wasser verteilt. Der Essigester wird nach Trocknen über $Na_2SO_4$ abdestilliert und der Rückstand zweimal mit Diethylether verrieben.

Ausbeute: 72,5g (114 %) Öl

1l. Fmoc-D-p-Cl-Phe-OH

118,0 g (519 mmol) H-D-p-Cl-Phe-OH werden in 2000 ml Dioxan/Wasser (1:1) suspendiert. Dazu gibt man 123 g $NaHCO_3$ (1464 mmol) und 164,5 g (488 mmol) Fmoc-ONSu. Der Ansatz rührt 5 Stunden bei Raumtemperatur und steht über Nacht bei dieser Temperatur. Der geringe Niederschlag wird abgesaugt, das Dioxan wird weitgehend abdestilliert, mit 2N HCl wird angesäuert (pH 2-3) und der Niederschlag abgesaugt und mit Wasser gut gewaschen. Aus 5 Liter Isopropanol wird die Substanz umkristallisiert. Nach 3-stündigem Stehen bei 4 °C wird abgesaugt und der Niederschlag mit Petrolether behandelt um das Isopropanol zu entfernen.

Ausbeute: 167,6 g (Charge I, enthält 0,76 % Fmoc-L-pCl-Phe-OH)
Schmp. 172-174 °C
$[\alpha]_D^{26}$ = +22,3° (c=1, in Dimethylformamid)

1m. Fmoc-D-p-Cl-Phe-D-Trp-Ser(tBu)-Tyr(tBu)-OtBu

72,5 g (ca. 100 mmol) H-D-Trp-Ser(tBu)-Tyr(tBu)-OtBu, 42,2 g (100 mmol) Fmoc-D-p-Cl-Phe-OH und 13,5 g (100 mmol) HOBt werden in 400 ml Dimethylformamid gelöst. Dazu gibt man bei 0 °C unter Rühren 20,6 g (100 mmol) DCC. Man läßt 1 Stunde bei 0 °C rühren und über Nacht bei Raumtemperatur stehen. Der DC-Harnstoff wird abgesaugt und das Filtrat eingeengt. Der Rückstand wird in Essigester gelöst, erneut filtriert und nacheinander mit Wasser, $NaHCO_3$-Lösung und Wasser extrahiert, über $Na_2SO_4$ getrocknet und eingeengt. Der Rückstand wird in Diethylether gelöst und mit Petrolether wieder amorph ausgefällt. Der Rückstand wird in 500 ml Methanol gelöst, filtriert und in 1,5 ml Wasser eingetropft. Der kristalline Niederschlag wird abgesaugt und über $P_2O_5$ im Hochvakuum getrocknet.

Ausbeute: 80 g (77,8 %)
$[\alpha]_D^{26}$ = +4,9° (c=1, in Methanol)
Schmp. 96-98 °C

1n. H-D-p-Cl-Phe-D-Trp-Ser(tBu)-Tyr(tBu)-OtBu

80 g (77,9 mmol) Fmoc-D-p-Cl-Phe-D-Trp-Ser(tBu)-Tyr(tBu)-OtBu werden in 600 ml Dimethylformamid gelöst. Dazu gibt man bei Raumtemperatur 81 ml Diethylamin. Nach 20 Minuten Reaktionszeit bei Raumtemperatur wird im Hochvakuum eingeengt und der Rückstand mit Petrolether verrieben. Dies wird zweimal wiederholt. Anschließend wird die Substanz in Diethylether gelöst, von Unlöslichem filtriert und eingeengt. Dieser Vorgang wird zweimal wiederholt.

Ausbeute 64,9 g Öl (104,9 %)

1o. Ac-D-Nal-D-p-Cl-Phe-D-Trp-Ser(tBu)-Tyr(tBu)-OtBu

64,9 g (ca. 77,9 mmol) H-D-p-Cl-Phe-D-Trp-Ser(tBu)-Tyr(tBu)-OtBu, 19,8 g (76,95 mmol) Ac-D-Nal-OH und 12,46 g (76,4 mmol) HOObt werden in 500 ml Dimethylformamid gelöst. Bei 0 °C gibt man unter Rühren 16,93 g (76,95 mmol) DCC zu. Man läßt 1 Stunde bei 0 °C rühren und über Nacht bei Raumtemperatur stehen. Danach wird der DC-Harnstoff abgesaugt, das Filtrat eingeengt und der Rückstand mit Essigester verrieben Der Niederschlag wird abgesaugt und getrocknet.

Ausbeute: 47,9 g (59 %)
Schmp. 224-228 °C unter Zersetzung
$[\alpha]_D^{26}$ = -12,6° (c=1, in 90 %iger Essigsäure)

1p. Ac-D-Nal-D-p-Cl-Phe-D-Trp-Ser-Tyr-OH

47,6 g (45,6 mmol) Ac-D-Nal-D-p-Cl-Phe-D-Trp-Ser(tBu)-Tyr(tBu)-OtBu werden in einer Mischung aus 350 ml 90 %iger wäßriger Trifluoressigsäure und 35 ml 1,2-Dimercaptoethan bei Raumtemperatur eingeengt und der Rückstand mit Diethylether verrieben und abgesaugt. Zur weiteren Reinigung wird die Substanz heiß in Isopropanol gelöst und mit Petrolether gefällt.

Ausbeute: 33,67 g,
Schmp. 196 °C unter Zersetzung (sintert ab 159 °C),
$[\alpha]_D^{26}$ = -3,5° (c=1, in Methanol)

1q. Ac-D-Nal-D-p-Cl-Phe-D-Trp-Ser-Tyr-D-Ser[Rha($Ac_3$)]-Leu-Ser[Rha($Ac_3$)]-Pro-Azagly-$NH_2$

Zu einer Lösung von 1 g (1 mmol) H-D-Ser[Rha($Ac_3$)]-Leu-Ser[Rha($Ac_3$)]-Pro-Azagly-$NH_2$, 875 mg Ac-D-Nal-D-p-Cl-Phe-D-Trp-Ser-Tyr-OH und 163 mg HOObt in 4 ml Dimethylformamid gibt man bei 0 °C unter Rühren 210 mg DCC. Nach einer Stunde bei 0 °C läßt man über Nacht bei Raumtemperatur stehen. Anderntags wird der Niederschlag abgesaugt und das Filtrat eingeengt. Der Rückstand wird eingeengt und mit MTB-Ether verrieben. Die Rohsubstanz wird an Kieselgel in Methylenchlorid/Methanol/Essigsäure/Wasser (9:1:0,1:0,1) chromatographiert.

Ausbeute: 1,09g (59 %)
$[\alpha]_D^{26}$ = -48.5° (c=1, in Eisessig)

1r. Ac-D-Nal-D-p-Cl-Phe-D-Trp-Ser-Tyr-D-Ser(Rha)-Leu-Ser(Rha)-Pro-Azagly-$NH_2$

Zu einer Lösung von 400 mg (0,21 mmol) Ac-D-Nal-D-p-Cl-Phe-D-Trp-Ser-Tyr-D-Ser[Rha($Ac_3$)]-Leu-Ser[Rha($Ac_3$)]-Pro-Azagly-$NH_2$ in 4 ml 90 %igem wäßrigem Methanol gibt man bei Raumtemperatur und unter Rühren 360 mg $K_2CO_3$. Man rührt 10 Minuten, säuert mit $KHSO_4$-Puffer an und extrahiert zweimal mit n-Pentanol. Die vereinigten n-Pentanol-Phasen werden eingeengt und an Kieselgel in n-Butanol/Eisessig/Wasser (63:9,5:27,5) durch Chromatographie gereinigt.

Ausbeute: 112 mg (27 %)
$[\alpha]_D^{26}$ = -38,2° (c=1, in Eisessig)

2. Beispiel

Ac-D-Nal-D-p-Cl-Phe-D-Pal-Ser-Tyr-D-Ser(Rha)-Leu-Ser(Rha)-Pro-Azagly-$NH_2$

2a. Fmoc-D-Pal-Ser(tBu)-Tyr(tBu)-OtBu

Zu einer Lösung von 3,88 g Fmoc-D-Pal-OH (10 mmol), 4,73 g HCl·H-Ser(tBu)-Tyr(tBu)-OtBu und 1,35 g HOBt in 40 ml Dimethylformamid gibt man bei 0 °C unter Rühren 1,3 ml Ethylmorpholin und 2,2 g DCC. Man rührt 1 Stunde bei 0 °C und läßt über Nacht bei Raumtemperatur stehen. Der Niederschlag wird abgesaugt und der Rückstand in Essigester gelöst. Nacheinander wird mit Wasser, gesättigter $NaHCO_3$-Lösung, $KHSO_4$-Puffer und Wasser extrahiert, über Natriumsulfat getrocknet und eingeengt.

Ausbeute: 3,74 g (46,3 %),
Schmp. 86-88 °C
$[\alpha]_D^{23}$ = +10,1° (c=1, in MeOH)

2b. H-D-Pal-Ser(tBu)-Tyr(tBu)-OtBu

Zu einer Lösung von 3,74 g Fmoc-D-Pal-Ser(tBu)-Tyr(tBu)-OtBu in 20 ml Dimethylformamid gibt man bei Raumtemperatur 4,8 ml Diethylamin. Nach 10 Minuten wird eingeengt und der Rückstand nach einander 2 mal mit Petrolether verrieben und in Diethylether gelöst. Anfallender Niederschlag wird abgesaugt und die Diethyletherlösung eingeengt.

Ausbeute: 2,7 g Öl

2c. Fmoc-D-p-Cl-Phe-D-Pal-Ser(tBu)-Tyr(tBu)-OtBu

Zu einer Lösung von obigem 2,7 g H-D-Pal-Ser(tBu)-Tyr(tBu)-OtBu, 2,16 g Fmoc-p-Cl-D-Phe-OH und 0,62 g HOBt in 30 ml Dimethylformamid gibt man bei 0 °C unter Rühren 1,02 g DCC. Man läßt 1 Stunde bei 0 °C rühren und über Nacht bei Raumtemperatur stehen. Der Niederschlag wird abgesaugt und das Filtrat eingeengt und der Rückstand wie bei Beispiel 2a aufgearbeitet. Der Rückstand wird aus Diethylether/Petrolether umgefällt.

Ausbeute: 3,25 g
Schmp. 98-102 °C
$[\alpha]_D^{23}$ = +12,0° (c=1, in MeOH)

2d. H-D-p-Cl-Phe-D-Pal-Ser(tBu)-Tyr(tBu)-OtBu

3,0 g Fmoc-D-p-Cl-Phe-D-Pal-Ser(tBu)-Tyr(tBu)-OtBu werden wie im Beispiel 2b mit 3,1 ml Diethylamin in 50 ml Dimethylformamid umgesetzt.

Ausbeute: 1,6 g Öl

2e. Ac-D-Nal-D-p-Cl-Phe-D-Pal-Ser(tBu)-Tyr(tBu)-OtBu

Zu einer Lösung von obigem 1,6 g H-D-p-Cl-Phe-D-Pal-Ser(tBu)-Tyr(tBu)-OtBu, 0,5 g Ac-D-Nal-OH und 0,32 g HOObt in 20 ml Dimethylformamid gibt man bei 0 °C und unter Rühren 0,43 g DCC. Man läßt 1 Stunde bei 0 °C rühren und über Nacht bei Raumtemperatur stehen. Der Niederschlag wird abgesaugt und das Filtrat eingeengt. Der Rückstand wird nach Verreiben mit Essigester abgesaugt.

Ausbeute: 1,43 g
Schmp. 208-210 °C
$[\alpha]_D^{23}$ = 7,0° (c=1, in Eisessig)

2f. Ac-D-Nal-D-p-Cl-Phe-D-Pal-Ser-Tyr-OH-trifluoracetat

1,4 g (1,39 mmol) Ac-D-Nal-D-p-Cl-Phe-D-Pal-Ser(tBu)-Tyr(tBu)-OtBu werden in eine Mischung aus 15 ml 90 %iger wäßriger Trifluoressigsäure und 1,5 ml 1,2-Dimercaptoethan eingetragen. Man läßt 1 Stunde bei Raumtemperatur stehen und engt ein. Der Rückstand wird mit Diethylether verrieben und abgesaugt.

Ausbeute: 1,1 g
Schmp. 284 °C (unter Zersetzung)

2g. Ac-D-Nal-D-p-Cl-Phe-D-Pal-Ser-Tyr-D-Ser[Rha($Ac_3$)]-Leu-Ser[Rha($Ac_3$)]-Pro-Azagly-$NH_2$

Zu einer Lösung von 1 g (1 mmol) H-D-Ser[Rha($Ac_3$)]-Leu-Ser[Rha($Ac_3$)]-Pro-Azagly-$NH_2$, 838 mg Ac-D-Nal-D-pCl-Phe-D-Pal-Ser-Tyr-OH-trifluoracetat und 163 mg HOObt in 4 ml Dimethylformamid gibt man 0,13 ml N-Ethylmorpholin und 210 mg DCC. Ansatz und Aufarbeitung wie bei Beispiel 1q, jedoch ohne Säulenreinigung.

Ausbeute an Rohsubstanz: 1,55 g (85 %)

2h. Ac-D-Nal-D-p-Cl-Phe-D-Pal-Ser-Tyr-D-Ser(Rha)-Leu-Ser(Rha)Pro-Azagly-$NH_2$

Zu einer Lösung von 1,5 g (0,8 mmol) des rohen Ac-D-Nal-D-p-Cl-Phe-D-Pal-Ser-Tyr-D-Ser[Rha($Ac_3$)]-Leu-Ser[Rha($Ac_3$)]-Pro-Azagly-$NH_2$ in 15 ml 90 %igem wäßrigem Methanol gibt man unter Rühren bei Raumtemperatur 1,4 g $K_2CO_3$. Man arbeitet wie im Beispiel 1r auf.

Das rohe Material wird an Kieselgel in Methylenchlorid/Methanol/Wasser/Essigsäure (20:4:1,5:5) und

Methanol/Wasser/Essigsäure (1:1:1) chromatographiert.
Ausbeute: 270 mg (22 %)
$[\alpha]_D^{26}$ = -45° (c=1, in Eisessig)

Beispiel 3

Ac-D-Nal-D-p-Cl-Phe-D-Trp-Ser-Tyr-D-Ser(Rha)-Leu-Ser(Xyl)-Pro-Azagly-$NH_2$

3a. H-Ser[Xyl($Ac_3$)]-OH

16,37 g Fmoc-Ser[Xyl($Ac_3$)]-OH werden in 100 ml Dimethylformamid analog Beispiel 1a mit 30,8 ml Dimethylamin umgesetzt.
Ausbeute: 9,65 g (95 %)

3b. Fmoc-Leu-Ser[Xyl($Ac_3$)]-OH

9,65 g (26,65 mmol) H-Ser[Xyl($Ac_3$)]-OH werden analog Beispiel 1b mit 12 g Fmoc-Leu-OObt in 70 ml Dimethylformamid umgesetzt.
Ausbeute: 12,8 g (69 %)

3c. Fmoc-Leu-Ser[Xyl($Ac_3$)]-Pro-Azagly-$NH_2$

12,8 g (18,65 mmol) Fmoc-Leu-Ser[Xyl($Ac_3$)]-OH werden analog Beispiei 1c mit 5,7 g $HClO_4 \cdot$H-Pro-Azagly-$NH_2$, 3,04 g HOObt, 2,36 ml N-Ethylmorpholin und 3,91 g DCC umgesetzt.
Ausbeute: 11,6 g (73 %)

3d. H-Leu-Ser[Xyl($Ac_3$)]-Pro-Azagly-$NH_2$

11,6 g (13,8 mmol) Fmoc-Leu-Ser[Xyl($Ac_3$)]-Pro-Azagly-$NH_2$ werden analog Beispiel 1d mit 15,2 ml Diethylamin in 100 ml Dimethylformamid umgesetzt.
Ausbeute: 7,47 g (86 %)

3e. Fmoc-D-Ser[Rha($Ac_3$)]-Leu-Ser[Xyl($Ac_3$)]-Pro-Azagly-$NH_2$

7,47 g H-Leu-Ser[Xyl($Ac_3$)]-Pro-Azagly-$NH_2$ werden analog Beispiel 1e mit 7,25 g Fmoc-D-Ser[Rha($Ac_3$)]-OH, 1,7 g HOBt und 2,54 g DCC umgesetzt.
Ausbeute: 10,48 g (71 %)
$[\alpha]_D^{26}$ = -57° (c=1, in Eisessig)

3f. H-D-Ser[Rha($AC_3$)]-Leu-Ser[Xyl($Ac_3$)]-Pro-Azagly-$NH_2$

2 g (1,65 mmol) Fmoc-D-Ser[Rha($Ac_3$)]-Leu-Ser[Xyl($Ac_3$)]-Pro-Azagly-$NH_2$ werden analog Beispiel 1f mit 1,8 ml Diethylamin in 15 ml Dimethylformamid umgesetzt.
Ausbeute: 1,47 g (90 %)
$[\alpha]_D^{26}$ = -68° (c=1, in Eisessig)

3g. Ac-D-Nal-D-p-Cl-Phe-D-Trp-Ser-Tyr-D-Ser(Rha)-Leu-Ser(Xyl)-Pro-Azagly-$NH_2$

990 mg (1 mmol) H-D-Ser[Rha($Ac_3$)]-Leu-Ser[Xyl($Ac_3$)]-Pro-Azagly-$NH_2$ werden analog Beispiel 1q mit 875 mg Ac-D-Nal-D-p-Cl-Phe-D-Trp-Ser-Tyr-OH, 163 mg HOObt und 210 mg DCC umgesetzt.
Ausbeute: 1,07 g (59 %)
$[\alpha]_D^{26}$ = -45° (c=1, in Eisessig)

3h. Ac-D-Nal-D-p-Cl-Phe-D-Trp-Ser-Tyr-D-Ser(Rha)-Leu-Ser(Xyl)-Pro-Azagly-$NH_2$

560 mg (0,3 mmol) Ac-D-Nal-D-p-Cl-Phe-D-Trp-Ser-Tyr-D-Ser[Rha($Ac_3$)]-Leu-Ser[Xyl($Ac_3$)]-Pro-Azagly-$NH_2$ werden analog Beispiel 1r mit 497 mg $K_2CO_3$ in 5 ml 90 %igem wäßrigem Methanol umgesetzt.
Ausbeute: 80 mg (26 %)
$[\alpha]_D^{26}$ = -39° (c=1, in Eisessig)

Beispiel 4

Ac-D-Nal-D-p-Cl-Phe-D-Pal-Ser-Tyr-D-Ser(Rha)-Leu-Ser(Fuc)-Pro-Azagly-$NH_2$

4a. H-Ser[Fuc($Ac_3$)]-OH

11,82 g (19,73 mmol) Fmoc-Ser[Fuc($Ac_3$)]-OH werden analog Beispiel 1a mit 21,7 ml Diethylamin in 50 ml Dimethylformamid umgesetzt.
Ausbeute: 7,08 g (95 %)

4b. Fmoc-Leu-Ser[Fuc($Ac_3$)]-OH

7,08 g (18,78 mmol) H-Ser[Fuc($Ac_3$)]-OH werden analog Beispiel 1b mit 8,41 g Fmoc-Leu-OObt in 100 ml Dimethylformamid umgesetzt.
Ausbeute: 8,23 g (68 %)

4c. Fmoc-Leu-Ser[Fuc($Ac_3$)]-Pro-Azagly-$NH_2$

8,2 g (11,51 mmol) Fmoc-Leu-Ser[Fuc($Ac_3$)]-OH werden analog Beispiel 1c mit 3,5 g $HClO_4 \cdot$H-Pro-

Azagly-$NH_2$, 1,88 g HOObt, 1,46 mol N-Ethylmorpholin und 2,42 g DCC in 60 ml Dimethylformamid umgesetzt.

Ausbeute: 6,87 g (69 %)

4d. H-Leu-Ser[Fuc($Ac_3$)]-Pro-Azagly-$NH_2$

6,87 g (7,93 mmol) Fmoc-Leu-Ser[Fuc($Ac_3$)]-Pro-Azagly-$NH_2$ werden analog Beispiel 1d mit 8,7 ml Diethylamin in 50 ml Dimethylformamid umgesetzt.

Ausbeute: 4,49 g (88 %)

4e. Fmoc-D-Ser[Rha($Ac_3$)]-Leu-Ser[Fuc($Ac_3$)]-Pro-Azagly-$NH_2$

4,49 g (6,97 mmol) H-Leu-Ser(Fuc)-Pro-Azagly-$NH_2$ werden analog Beispiel 1e mit 4,18 g Fmoc-D-Ser[Rha($Ac_3$)]-OH, 976 mg HOBt und 1,47 g DCC in 40 ml Dimethylformamid umgesetzt.

Ausbeute: 6,64 g (77 %)

$[\alpha]_D^{26}$ = -23,3° (c=1, in Eisessig)

4f. H-D-Ser[Rha($Ac_3$)]-Leu-Ser[Fuc($Ac_3$)]-Pro-Azagly-$NH_2$

6,6 g (5,38 mmol) Fmoc-D-Ser[Rha($Ac_3$)]-Leu-Ser[Fuc($Ac_3$)]-Pro-Azagly-$NH_2$ werden analog Beispiel 1f mit 6 ml Diethylamin in 20 ml Dimethylformamid umgesetzt.

Ausbeute: 4,82 g (90 %)

$[\alpha]_D^{26}$ = -45° (c=1, in Eisessig)

4g. Ac-D-Nal-D-p-Cl-Phe-D-Pal-Ser-Tyr-D-Ser[Rha($Ac_3$)]-Leu-Ser[Fuc($Ac_3$)]-Pro-Azagly-$NH_2$

1g (1 mmol) H-D-Ser[Rha($Ac_3$)]-Leu-Ser[Fuc($Ac_3$)]-Pro-Azagly-$NH_2$ werden analog Beispiel 2g mit 838 mg Ac-D-Nal-D-p-Cl-Phe-D-Pal-Ser-Tyr-OH-trifluoracetat, 0,13 ml N-Ethylmorpholin, 163 mg HOObt und 210 mg DCC in 4 ml Dimethylformamid umgesetzt.

Ausbeute: 1,35 g (74 %)

4h. Ac-D-Nal-D-p-Cl-Phe-D-Pal-Ser-Tyr-D-Ser(Rha)-Leu-Ser(Fuc)-Pro-Azagly-$NH_2$

1,35 g Ac-D-Nal-D-p-Cl-Phe-D-Pal-Ser-Tyr-D-Ser[Rha($Ac_3$)]-Leu-Ser[Fuc($Ac_3$)]-Pro-Azagly-$NH_2$ werden analog Beispiel 2h mit 1,24 g $K_2CO_3$ in 15 ml 90 %igem Methanol umgesetzt.

Ausbeute: 170mg (15 %)

$[\alpha]_D^{26}$ = -17,3° (c=1, in Eisessig)

Beispiel 5

Ac-D-Nal-D-p-Cl-Phe-D-Pal-Ser-Tyr-D-Ser(Rha)-Leu-Ser(Rha)-Pro-D-Ala-$NH_2$

5a. Fmoc-Pro-D-Ala-$NH_2$

Zu einer Lösung von 5,4 g (16 mmol) Fmoc-Pro-OH, 2g HCl·H-D-Ala-$NH_2$ und 2,24 g HOBt in 30 ml Dimethylformamid gibt man bei 0 °C unter Rühren 3,36 g DCC. Man läßt 1 Stunde bei 0 °C rühren und über Nacht bei Raumtemperatur stehen. Der Niederschlag wird abgesaugt und das Filtrat eingeengt. Der Rückstand wird in Essigester gelöst und nacheinander mit Wasser, 2 mal mit gesättigter $NaHCO_3$-Lösung, Wasser, $KHSO_4$-Puffer und Wasser ausgeschüttelt. Hierbei fällt bereits Fmoc-Pro-D-Ala-$NH_2$ in der Essigester-Phase aus und wird abgesaugt. Die Essigestermutterlauge wird eingeengt und mit Diethylether verrieben und abgesaugt.

vereinigte Ausbeute: 5,76 g (88 %)

$[\alpha]_D^{26}$ = -29° (c=1, in Essigester)

5b. H-Pro-D-Ala-$NH_2$

Zu einer Lösung von 5,66 g (13,9 mmol) Fmoc-Pro-D-Ala-$NH_2$ in 30 ml Dimethylformamid gibt man bei Raumtemperatur 15,3 ml Diethylamin. Nach 6 Minuten wird die Lösung im Hochvakuum eingeengt. Der Rückstand wird mit Diethylether verrieben.

Ausbeute: 2,47 g (96 %)

5c. Fmoc-Leu-Ser[Rha($Ac_3$)]-Pro-D-Ala-$NH_2$

Zu einer Lösung von 9,5 g (13,35 mmol) Fmoc-Leu-Ser[Rha($Ac_3$)]-OH, 2,4 g H-Pro-D-Ala-$NH_2$ und 2,17 g HOObt gibt man bei 0 °C unter Rühren 2,8 g DCC.

Man läßt 1 Stunde bei 0 °C rühren und über Nacht bei Raumtemperatur stehen. Der Niederschlag wird abgesaugt und das Filtrat eingeengt. Der Rückstand wird in einer Mischung aus Essigester/Pentanol (10:2) gelöst und nacheinander mit Wasser, 2 mal mit gesättigter $NaHCO_3$-Lösung, Wasser, $KHSO_4$-Puffer und Wasser gewaschen. Die organische Phase wird über $Na_2SO_4$ getrocknet und eingeengt. Der Rückstand wird aus Diethylether/Petrolether gefällt und abgesaugt.

Ausbeute: 7,75 g (66 %)

$[\alpha]_D^{26}$ = -38° (c=1, in Eisessig)

5d. H-Leu-Ser[Rha($Ac_3$)]-Pro-D-Ala-$NH_2$

7,7 g Fmoc-Leu-Ser[Rha(Ac$_3$)]-Pro-D-Ala-NH$_2$ werden analog Beispiel 1d mit 9,7 ml Diethylamin in 30 ml Dimethylformamid umgesetzt.

Ausbeute: 5,25 g (90 %)

5e. Fmoc-D-Ser[Rha(Ac$_3$)]-Leu-Ser[Rha(Ac$_3$)]-Pro-D-Ala-NH$_2$

5,2 g (8,8 mmol) Fmoc-D-Ser[Rha(Ac$_3$)]-OH werden analog Beispiel 1e mit 5,25 g H-Leu-Ser[Rha(Ac$_3$)]-Pro-D-Ala-NH$_2$, 1,23 g HOBt und 1,84 g DCC in 50 ml Dimethylformamid umgesetzt.

Ausbeute: 8,01 g (73 %)

$[\alpha]_D^{26}$ = -43,3° (c=1, in Eisessig)

5f. H-D-Ser[Rha(Ac$_3$)]-Leu-Ser[Rha(Ac$_3$)]-Pro-D-Ala-NH$_2$

4 g (3,23 mmol) Fmoc-D-Ser[Rha(Ac$_3$)]-Leu-Ser[Rha(Ac$_3$)]-Pro-D-Ala-NH$_2$ werden analog Beispiel 1f mit 3,6 ml Diethylamin in 10 ml Dimethylformamid umgesetzt.

Ausbeute: 2,98 g (91 %)

$[\alpha]_D^{26}$ = -49° (c=1, in Eisessig)

5g. Ac-D-Nal-D-p-Cl-Phe-D-Pal-Ser-Tyr-D-Ser[Rha(Ac$_3$)]-Leu-Ser[Rha(Ac$_3$)]-Pro-D-Ala-NH$_2$

838 mg (1 mmol) Ac-D-Nal-D-p-Cl-Phe-D-Pal-Ser-Tyr-OH-trifluoracetat werden analog Beispiel 2g mit 1 g H-D-Ser[Rha(Ac$_3$)]-Leu-Ser[Rha(Ac$_3$)]-Pro-D-Ala-NH$_2$, 163 mg HOObt, 0,13 ml N-Ethylmorpholin und 210 mg DCC in 9 ml Dimethylformamid umgesetzt.

Ausbeute: 1,36 g (74 %)

$[\alpha]_D^{26}$ = -25° (c=1, in Eisessig)

5h. Ac-D-Nal-D-p-Cl-Phe-D-Pal-Ser-Tyr-D-Ser(Rha)-Leu-Ser(Rha)-Pro-D-Ala-NH$_2$

1,34 g (0,74 mmol) Ac-D-Nal-D-p-Cl-Phe-D-Pal-Ser-Tyr-D-Ser[Rha(Ac$_3$)]-Leu-Ser[Rha(Ac$_3$)]-Pro-D-Ala-NH$_2$ werden analog Beispiel 2h mit 1,24 g $K_2CO_3$ in 15 ml 90 %igem wäßrigem Methanol umgesetzt.

Ausbeute: 230 mg (20 %

$[\alpha]_D^{26}$ = -36° (c=1, in Eisessig)

## Patentansprüche

### Patentansprüche für folgende Vertragsstaaten : AT, BE, CH, DE, DK, FR, GB, IT, LI, LU, NL, SE

**1.** Peptid der allgemeinen Formel I,

$$\overset{1}{X} - \overset{}{A} - ...$$

in welcher

X (C$_2$-C$_8$)-Alkanoyl bedeutet;

A D-Nal (2), D-Phe oder D-Trp bedeutet, wobei der Aromat gegebenenfalls durch einen oder zwei gleiche oder verschiedene Reste aus der Reihe Brom, Chlor, Fluor, Nitro, C$_1$-C$_4$-Alkyl und C$_1$-C$_4$-Alkoxy substituiert sein kann;

B D-Phe bedeutet, das gegebenenfalls durch einen oder zwei gleiche oder verschiedene Reste aus der Reihe Brom, Chlor, Fluor, Nitro, Amino, C$_1$-C$_4$-Alkyl, und C$_1$-C$_4$-Alkoxy substituiert sein kann;

C D-Pal (3), D-Phe oder D-Trp bedeutet, wobei der Aromat von D-Phe und D-Trp gegebenenfalls durch einen oder zwei gleiche oder verschiedene Reste aus der Reihe Brom, Chlor, Fluor, Nitro, C$_1$-C$_4$-Alkyl und C$_1$-C$_4$-Alkoxy substituiert sein kann;

D Tyr oder His bedeutet;

E D-Ser(R$^1$) bedeutet;

F Leu, Trp oder Phe bedeutet;

G L-Ser(R$^1$) bedeutet;

H Gly-NH$_2$, D-Ala-NH$_2$ oder Azagly-NH$_2$ bedeutet;

R$^1$ einen Glycosylrest bedeutet;

oder dessen physiologisch verträgliches Salz.

**2.** Peptid der allgemeinen Formel 1, gemäß Anspruch 1, in welcher

X Acetyl;

A D-Nal(2);

B D-Phe(p-Cl);
C D-Pal(3) oder D-Trp;
D Tyr;
E D-Ser($R^1$);
F Leu;
G L-Ser($R^1$);
H D-Ala-$NH_2$ oder Azagly-$NH_2$ bedeuten,
oder dessen physiologisch verträgliches Salz.

**3.** Verfahren zur Herstellung eines Peptids der allgemeinen Formel I gemäß einem oder mehreren der Ansprüche 1 bis 2, dadurch gekennzeichnet, daß man ein Fragment mit N-terminaler freier Aminogruppe mit einem Fragment mit C-terminaler freier Carboxylgruppe kondensiert, eine oder mehrere zum Schutz funktioneller Gruppen gegebenenfalls temporär eingeführte Schutzgruppen abspaltet und das so erhaltene Peptid gegebenenfalls in sein physiologisch verträgliches Salz überführt.

**4.** Peptid der allgemeinen Formel I gemäß einem oder mehreren Ansprüche 1 bis 2 zur Anwendung als Heilmittel.

**5.** Peptid der allgemeinen Formel I gemäß einem oder mehreren der Ansprüche 1 bis 2 zur Anwendung als Plasma-Gonadotropin, -Testosteron und -Östrogen senkendes Mittel.

**6.** Pharmazeutische Zubereitung enthaltend ein Peptide der allgemeinen Formel I gemäß einem oder mehreren der Ansprüche 1 bis 2 oder dessen physiologisch verträgliches Salz.

**7.** Verfahren zur Herstellung einer Zubereitung gemäß Anspruch 6, dadurch gekennzeichnert, daß man ein Peptid der allgemeinen Formel I oder dessen physiologisch verträgliches Salz zusammen mit einem physiologisch verträglichen Träger und gegebenenfalls weiteren Zusatz- Hilfs- und/oder Konservierungsstoffen in eine geeignete Darreichungsform bringt.

**Patentansprüche für folgende Vertragsstaaten : ES, GR**

**1.** Verfahren zur Herstellung eines Peptids der allgemeinen Formel I,

$$\overset{}{X}-\overset{1}{A}-\overset{2}{B}-\overset{3}{C}-\overset{4}{Ser}-\overset{5}{D}-\overset{6}{E}-\overset{7}{F}-\overset{8}{G}-\overset{9}{Pro}-\overset{10}{H} \quad (I),$$

in welcher
X ($C_2$-$C_8$)-Alkanoyl bedeutet;
A D-Nal (2), D-Phe oder D-Trp bedeutet, wobei der Aromat gegebenenfalls durch einen oder zwei gleiche oder verschiedene Reste aus der Reihe Brom, Chlor, Fluor, Nitro, $C_1$-$C_4$-Alkyl und $C_1$-$C_4$-Alkoxy substituiert sein kann;
B D-Phe bedeutet, das gegebenenfalls durch einen oder zwei gleiche oder verschiedene Reste aus der Reihe Brom, Chlor, Fluor, Nitro, Amino, $C_1$-$C_4$-Alkyl, und $C_1$-$C_4$-Alkoxy substituiert sein kann;
C D-Pal (3), D-Phe oder D-Trp bedeutet, wobei der Aromat von D-Phe und D-Trp gegebenenfalls durch einen oder zwei gleiche oder verschiedene Reste aus der Reihe Brom, Chlor, Fluor, Nitro, $C_1$-$C_4$-Alkyl und $C_1$-$C_4$-Alkoxy substituiert sein kann;
D Tyr oder His bedeutet;
E D-Ser($R^1$) bedeutet;
F Leu, Trp oder Phe bedeutet;
G L-Ser($R^1$) bedeutet;
H Gly-$NH_2$, D-Ala-$NH_2$ oder Azagly-$NH_2$ bedeutet;
$R^1$ einen Glycosylrest bedeutet;
oder dessen physiologisch verträgliches Salz, dadurch gekennzeichnet, daß man ein Fragment mit N-terminaler freier Aminogruppe mit einem Fragment mit C-terminaler freier Carboxylgruppe kondensiert, eine oder mehrere zum Schutz fuktioneller Gruppen gegebenenfalls temporär eingeführte Schutzgruppen abspaltet und das so erhaltene Peptid gegebenenfalls in sein physiologisch verträgliches Salz überführt.

2. Verfahren gemäß Anspruch 1, in welcher
   X Acetyl;
   A D-Nal(2);
   B D-Phe(p-Cl);
   C D-Pal(3) oder D-Trp;
   D Tyr;
   E D-Ser($R^1$);
   F Leu;
   G L-Ser($R^1$);
   H D-Ala-$NH_2$ oder Azagly-$NH_2$ bedeuten,
   oder dessen physiologisch verträgliches Salz.

3. Verfahren zur Herstellung einer pharmazeutischen Zubereitung enthaltend ein Peptid der allgemeinen Formel I gemäß einem oder mehreren der Ansprüche 1 bis 2 oder dessen physiologisch verträgliches Salz, dadurch gekennzeichnet, daß man ein Peptid der allgemeinen Formel I oder deren pysiologisch verträgliches Salz zusammen mit einem physiologisch verträglichen Träger und gegebenenfalls weiteren Zusatz- und/oder Konservierungsstoffen in eine geeignete Darreichungsform bringt.

## Claims

**Claims for the following Contracting States : AT, BE, CH, DE, DK, FR, GB, IT, LI, LU, NL, SE**

1. A peptide of the formula I

   1 2 3 4 5 6 7 8 9 10
   X - A - B - C - Ser - D - E - F - G - Pro - H (I),

   in which
   X is $C_2$-$C_8$-alkanoyl;
   A is D-Nal (2), D-Phe or D-Trp, where the aromatic ring can optionally be substituted by one or two identical or different radicals from the series comprising bromine, chlorine, fluorine, nitro, $C_1$-$C_4$-alkyl and $C_1$-$C_4$-alkoxy;
   B is D-Phe which can optionally be substituted by one or two identical or different radicals from the series comprising bromine, chlorine, fluorine, nitro, amino, $C_1$-$C_4$-alkyl and $C_1$-$C_4$-alkoxy;
   C is D-Pal (3), D-Phe or D-Trp, where the aromatic ring of D-Phe and D-Trp can optionally be substituted by one or two identical or different radicals from the series comprising bromine, chlorine, fluorine, nitro, $C_1$-$C_4$-alkyl and $C_1$-$C_4$-alkoxy;
   D is Tyr or His;
   E is D-Ser($R^1$);
   F is Leu, Trp or Phe;
   G is L-Ser($R^1$);
   H is Gly-$NH_2$, D-Ala-$NH_2$ or Azagly-$NH_2$;
   $R^1$ is a glycosyl radical;
   or the physiologically tolerated salt thereof.

2. A peptide of the formula I as claimed in claim 1, in which
   X is acetyl;
   A is D-Nal(2);
   B is D-Phe(p-Cl);
   C is D-Pal(3) or D-Trp;
   D is Tyr;
   E is D-Ser($R^1$);
   F is Leu;
   G is D-Ala-$NH_2$ or Azagly-$NH_2$,
   or the physiologically tolerated salt thereof.

3. A process for the preparation of a peptide of the formula I as claimed in one or more of claims 1 to 2, which comprises condensing a fragment with an N-terminal free amino group with a fragment with a C-terminal free carboxyl group, eliminating one or more protective groups which may have been temporarily introduced to protect functional groups, and converting the peptide obtained in this way where appropriate into its physiologically tolerated salt.

4. A peptide of the formula I as claimed in one or more of claims 1 to 2 for use as medicine.

5. A peptide of the formula I as claimed in one or more of claims 1 to 2 for use as agent lowering plasma gonadotropin, testosterone and estrogen.

6. A pharmaceutical composition containing a peptide of the formula I as claimed in one or more of claims 1 to 2 or the physiologically tolerated salt thereof.

7. A process for the preparation of a composition as claimed in claim 6, which comprises converting a peptide of the formula I or the physiologically tolerated salt thereof, together with a physiologically tolerated excipient and, where appropriate, further additives, auxiliaries and/or preservatives, into a suitable dosage form.

**Claims for the following Contracting States : ES, GR**

1. A process for the preparation of a peptide of the formula I

$$X - \overset{1}{A} - \overset{2}{B} - \overset{3}{C} - \overset{4}{Ser} - \overset{5}{D} - \overset{6}{E} - \overset{7}{F} - \overset{8}{G} - \overset{9}{Pro} - \overset{10}{H} \quad (I),$$

in which

X is $C_2$-$C_8$-alkanoyl;
A is D-Nal (2), D-Phe or D-Trp, where the aromatic ring can optionally be substituted by one or two identical or different radicals from the series comprising bromine, chlorine, fluorine, nitro, $C_1$-$C_4$-alkyl and $C_1$-$C_4$-alkoxy;
B is D-Phe which can optionally be substituted by one or two identical or different radicals from the series comprising bromine, chlorine, fluorine, nitro, amino, $C_1$-$C_4$-alkyl and $C_1$-$C_4$-alkoxy;
C is D-Pal (3), D-Phe or D-Trp, where the aromatic ring of D-Phe and D-Trp can optionally be substituted by one or two identical or different radicals from the series comprising bromine, chlorine, fluorine, nitro, $C_1$-$C_4$-alkyl and $C_1$-$C_4$-alkoxy;
D is Tyr or His;
E is D-Ser($R^1$);
F is Leu, Trp or Phe;
G is L-Ser($R^1$);
H is Gly-$NH_2$, D-Ala-$NH_2$ or Azagly-$NH_2$;
$R^1$ is a glycosyl radical;

or the physiologically tolerated salt thereof, which comprises condensing a fragment with an N-terminal free amino group with a fragment with a C-terminal free carboxyl group, eliminating one or more protective groups which may have been tempora-rily introduced to protect functional groups, and converting the peptide obtained in this way where appropriate into its physiologically tolerated salt.

2. The process as claimed in claim 1, in which

X is acetyl;
A is D-Nal(2);
B is D-Phe(p-Cl);
C is D-Pal(3) or D-Trp;
D is Tyr;
E is D-Ser($R^1$);
F is Leu;
G is D-Ala-$NH_2$ or Azagly-$NH_2$,

or the physiologically tolerated salt thereof.

3. A process for the preparation of a pharmaceutical composition containing a peptide of the formula I as defined in one or more of claims 1 to 2 or the physiologically tolerated salt thereof, which comprises converting a peptide of the formula I or the physiologically tolerated salt thereof, together with a physiologically tolerated excipient and, where appropriate, further additives and/or preservatives, into a suitable dosage form.

## Revendications

### Revendications pour les Etats contractants suivants : AT, BE, CH, DE, DK, FR, GB, IT, LI, LU, NL, SE

1. Peptide de formule générale I:

```
1 2 3 4    5 6 7 8 9    10
X-A-B-C-Ser-D-E-F-G-Pro-H          (I),
```

dans laquelle
X représente un groupe alcanoyle en $C_{2-8}$,
A représente D-Nal(2), D-Phe ou D-Trp, le radical aromatique pouvant être éventuellement substitué par un ou deux substituants identiques ou différents pris parmi bromo, chloro, fluoro, nitro, alkyle en $C_{1-4}$, et alcoxy en $C_{1-4}$,
B représente D-Phe, qui peut éventuellement être substitué par un ou deux substituants identiques ou différents pris parmi bromo, chloro, fluoro, nitro, amino, alkyle en $C_{1-4}$, et alcoxy en $C_{1-4}$,
C représente D-Pal(3), D-Phe ou D-Trp, le radical aromatique de D-Phe ou D-Trp pouvant être éventuellement substitué par un ou deux substituants identiques ou différents pris parmi bromo, chloro, fluoro, nitro, alkyle en $C_{1-4}$, et alcoxy en $C_1$-$_4$,
D représente Tyr ou His,
E représente D-Ser($R^1$),
F représente Leu, Trp ou Phe,
G représente L-Ser($R^1$),
H représente Gly-$NH_2$, D-Ala-$NH_2$ ou Azagly-$NH_2$,
$R^1$ représente un radical glycosyle,
ou un de ses sels physiologiquement acceptables.

2. Peptide de formule générale I, selon la revendication 1, dans laquelle
X est acétyle,
A est D-Nal(2),
B est D-Phe(p-Cl),
C est D-Pal(3) ou D-Trp,
D est Tyr,
E est D-Ser($R^1$),
F est Leu,
G est L-Ser($R^1$),
H est D-Ala-$NH_2$ ou Azagly-$NH_2$,
ou un de ses sels physiologiquement acceptables.

3. Procédé de préparation d'un peptide de formule générale I selon une ou plusieurs des revendications 1 à 2, caractérisé en ce qu'on condense un fragment à groupe amino N-terminal libre avec un fragment à groupe carboxyle C-terminal libre, on détache un ou plusieurs groupe(s) protecteur(s), qui peuvent avoir été temporairement introduit(s) pour protéger les groupes fonctionnels, et on transforme éventuellement le peptide ainsi obtenu en son sel physiologiquement acceptable.

4. Peptide de formule générale I selon une ou plusieurs des revendications 1 à 2, utilisé comme médicament.

5. Peptide de formule générale I selon une ou plusieurs des revendications 1 à 2, utilisé comme médicament abaisseur du taux de gonadotropine, de testostérone et d'oestrogène plasmatiques.

6. Préparation pharmaceutique contenant un peptide de formule générale I selon une ou plusieurs des revendications 1 à 2 ou un de ses sels physiologiquement acceptables.

7. Procédé d'obtention d'une préparation selon la revendication 6, caractérisé en ce qu'on met sous une forme d'administration appropriée un peptide de formule générale I ou un de ses sels physiologiquement acceptables, conjointement avec un véhicule physiologiquement compatible et éventuellement avec d'autres additifs, adjuvants et/ou conservateurs.

**Revendications pour les Etats contractants suivants : ES, GR**

1. Procédé de préparation d'un peptide de formule générale I:

$$\overset{1}{X}-\overset{2}{A}-\overset{3}{B}-\overset{4}{C}-\overset{5}{Ser}-\overset{6}{D}-\overset{7}{E}-\overset{8}{F}-\overset{9}{G}-\overset{10}{Pro}-H \qquad (I),$$

dans laquelle
X représente un groupe alcanoyle en $C_{2-8}$,
A représente D-Nal(2), D-Phe ou D-Trp, le radical aromatique pouvant être éventuellement substitué par un ou deux substituants identiques ou différents pris parmi bromo, chloro, fluoro, nitro, alkyle en $C_{1-4}$, et alcoxy en $C_{1-4}$,
B représente D-Phe, qui peut éventuellement être substitué par un ou deux substituants identiques ou différents pris parmi bromo, chloro, fluoro, nitro, amino, alkyle en $C_{1-4}$, et alcoxy en $C_{1-4}$,
C représente D-Pal(3), D-Phe ou D-Trp, le radical aromatique de D-Phe ou D-Trp pouvant être éventuellement substitué par un ou deux substituants identiques ou différents pris parmi bromo, chloro, fluoro, nitro, alkyle en $C_{1-4}$, et alcoxy en $C_{1}$-$_{4}$,
D représente Tyr ou His,
E représente D-Ser($R^1$),
F représente Leu, Trp ou Phe,
G représente L-Ser($R^1$),
H représente Gly-$NH_2$, D-Ala-$NH_2$ ou Azagly-$NH_2$,
$R^1$ représente un radical glycosyle,
ou d'un de ses sels physiologiquement acceptables, caractérisé en ce qu'on condense un fragment à groupe amino N-terminal libre avec un fragment à groupe carboxyle C-terminal libre, on détache un ou plusieurs groupe(s) protecteur(s), qui peuvent avoir été temporairement introduit(s) pour protéger les groupes fonctionnels, et on transforme éventuellement le peptide ainsi obtenu en son sel physiologiquement acceptable.

2. Procédé de préparation d'un peptide de formule générale I selon la revendication 1, dans laquelle
X est acétyle,
A est D-Nal(2),
B est D-Phe(p-Cl),
C est D-Pal(3) ou D-Trp,
D est Tyr,
E est D-Ser($R^1$),
F est Leu,
G est L-Ser($R^1$),
H est D-Ala-$NH_2$ ou Azagly-$NH_2$,
ou d'un de ses sels physiologiquement acceptables.

3. Procédé d'obtention d'une préparation pharmaceutique contenant un peptide de formule générale I selon une ou plusieurs des revendications 1 à 2 ou un de ses sels physiologiquement acceptables, caractérisé en ce qu'on met sous une forme d'administration appropriée un peptide de formule générale I ou un de ses sels physiologiquement acceptables, conjointement avec un véhicule physiologiquement compatible et éventuellement avec d'autres additifs et/ou conservateurs.